# EUROPEAN PATENT SPECIFICATION

(11) **EP 3 318 213 B1**
(45) Date of publication and mention of the grant of the patent: **12.10.2022**
(21) Application number: 17199690.3
(22) Date of filing: 02.11.2017
(51) Int. Cl.: A61B 34/30, A61B 5/06, A61B 17/17, A61B 17/34, A61B 34/20, A61B 34/10

(54) **SYSTEM FOR MEASURING DEPTH OF INSTRUMENTATION**
SYSTEM ZUR MESSUNG DER INSTRUMENTIERUNGSTIEFE
SYSTÈME DE MESURE DE PROFONDEUR D'INSTRUMENTATION

(30) Priority: 04.11.2016 US 201615343255
(43) Date of publication of application: 09.05.2018
(73) Proprietor: Globus Medical, Inc, Audubon, PA 19403 (US)
(72) Inventor: CRAWFORD, Neil R., Chandler, AZ 85224 (US); MAJOR, Chris, Reisterstown, MD 21113 (US)
(74) Representative: Morabito, Sara

(56) References cited:
- WO-A1-2013/192598
- WO-A1-2015/023665
- US-A1- 2012 289 820
- US-A1- 2014 135 744
- US-A1- 2014 221 822
- US-A1- 2014 275 955

## Description

### FIELD OF THE INVENTION

The present disclosure relates to position recognition systems, and in particular, position recognition systems that allow measurement of instrumentation and tools in a patient using robot-assisted surgical techniques.

### BACKGROUND OF THE INVENTION

Various medical procedures require the accurate localization of a three-dimensional position of a surgical instrument within the body in order to effect optimized treatment. For example, some surgical procedures to fuse vertebrae require that a surgeon drill multiple holes into the bone structure at specific locations. To achieve high levels of mechanical integrity in the fusing system, and to balance the forces created in the bone structure, it is necessary that the holes are drilled at the correct location. Vertebrae, like most bone structures, have complex shapes including non-planar curved surfaces making accurate and perpendicular drilling difficult. Conventionally, a surgeon manually holds and positions a drill guide tube by using a guidance system to overlay the drill tube's position onto a three dimensional image of the bone structure. This manual process is both tedious and time consuming. The success of the surgery is largely dependent upon the dexterity of the surgeon who performs it.

When a surgeon performs minimally invasive surgery and prepares to place a screw into bone, it is often desirable to insert a dilator tube from the surface of the skin through muscle and connective tissue down to a position where the distal end of the dilator tube is adjacent to bone. The dilator tube serves as a corridor through which drilling and other surgical steps can occur. Currently, the most frequently used method for gauging whether the dilator is sufficiently far enough inserted is to record x-ray images, which is effective but exposes the patient and surgical staff to x-rays and can be time consuming. If image guidance were to be used instead of x-rays, it would spare the patient and staff some exposure to x-rays. However, a tracking array mounted on a dilator tube has the drawbacks that it is unwieldy and obtrusive to the surgeon in an area where a great deal of surgical activity is occurring.

Thus, there is a need to be able to measure the depth of surgical instrumentation in a manner that limits the exposure of the patient and medical staff to unnecessary radiation from imaging systems without obstructing and impeding a medical staff's ability to perform the surgical operation on the patient. The present disclosure overcomes the disadvantages of current traditional surgical techniques and robot-assisted surgical techniques. For example, using known positions of components of a robot surgical system allows a surgeon to position a long shaft such as a dilator tube within a secondary tracked tube, such as the robot's end effector guide tube or a free tracked external guide tube, to a depth such that the dilator tube contacts the target bony structure. This positioning would allow tracking the dilator tube indirectly without tracking the dilator itself and without the use of x-ray images as it is further inserted into a patient.

WO 2013/192598 A1, US 2014/275955 A1, US 2012/289820 A1, US 2014/221822 A1, US 2014/135744 A1, and WO 2015/023665 A1 represent relevant prior art.

### SUMMARY OF THE INVENTION

To meet this and other needs, devices, systems, and methods for determining the distance or depth for a surgical instrument to contact a target bone of a patient during robot assisted surgery is provided.

The invention is as defined by independent claim 1. Dependent claims disclose preferred embodiments.

According to one exemplary embodiment, a surgical robot system may be configured to determine a distance for a surgical instrument to contact a target bone of a patient during a surgical operation. The system according to the invention includes a guide tube comprising a tracking marker and wherein the guide tube is configured to receive the surgical instrument, a tracking subsystem having a position sensor that recognizes the tracking marker in a navigational space, a platform interface module that is configured to receive a signal from the tracking subsystem indicative of a position of the guide tube based on the tracking marker, and a computer subsystem, including a computer and a display, that is configured to receive a first viewplane scan of the target bone and a second viewplane scan of the target bone. The first viewplane and the second viewplane form an intersection of views of the target bone. The computer subsystem is also configured to receive the position of the guide tube from the platform interface module and depicts the tracked guide tube and a vector extending from the tracked guide tube indicative of the distance to contact the target bone of the patient, relative to a distal portion of the guide tube, on both the first viewplane scan and the second viewplane scan, and wherein the vector represents a central axis of the guide tube being coincident to the intersection.

According to an example not according to the invention, a method for determining the distance for a surgical instrument to contact a target bone of a patient during a surgical operating using a robotic surgical system may be provided. The method may include receiving, by a computer subsystem having a computer and a display, a first viewplane scan of the target bone and a second viewplane scan of the target bone, wherein the first viewplane and the second viewplane form an intersection of views of the target bone in an image space. The method may also include receiving, by the computer subsystem, a position of a guide tube in navigational space, and determining, by the computer, a distance between a distal portion of the guide tube to the target bone of the patient, wherein the distance may be determined such that a central axis of the guide tube is coincident to the intersection. The method may also include depicting, via the display, a vector indicative of the distance to contact the target bone of the patient on at least one of the first viewplane scan and the second viewplane scan.

According to another unclaimed aspect it is provided a method for determining the distance for a surgical instrument to contact a target bone of a patient during a surgical operating using a robotic surgical system, said method comprising: receiving, by a computer subsystem having a computer and a display, a first viewplane scan of the target bone and a second viewplane scan of the target bone, wherein the first viewplane and the second viewplane form an intersection of views of the target bone in an image space; receiving, by the computer subsystem, a position of a guide tube in navigational space; determining, by the computer, a distance between a distal portion of the guide tube to the target bone of the patient, wherein the distance is determined such that a central axis of the guide tube is coincident to the intersection; and depicting, via the display, a vector indicative of the distance to contact the target bone of the patient on at least one of the first viewplane scan and the second viewplane scan, wherein the surgical instrument received in the guide tube.

In a version, the surgical instrument contains graduated markings indicating the actual depth of the surgical instrument past the distal portion of the guide tube.

According to another unclaimed aspect it is provided a method for determining the distance for a surgical instrument to contact a target bone of a patient during a surgical operating using a robotic surgical system, said method comprising: receiving, by a computer subsystem having a computer and a display, a first viewplane scan of the target bone and a second viewplane scan of the target bone, wherein the first viewplane and the second viewplane form an intersection of views of the target bone in an image space; receiving, by the computer subsystem, a position of a guide tube in navigational space; determining, by the computer, a distance between a distal portion of the guide tube to the target bone of the patient, wherein the distance is determined such that a central axis of the guide tube is coincident to the intersection; and depicting, via the display, a vector indicative of the distance to contact the target bone of the patient on at least one of the first viewplane scan and the second viewplane scan, wherein the first viewplane is a sagittal view plane with respect to the target bone and the second viewplane is an axial viewplane with respect to the target bone.

According to another unclaimed aspect it is provided a method for determining the distance for a surgical instrument to contact a target bone of a patient during a surgical operating using a robotic surgical system, said method comprising: receiving, by a computer subsystem having a computer and a display, a first viewplane scan of the target bone and a second viewplane scan of the target bone, wherein the first viewplane and the second viewplane form an intersection of views of the target bone in an image space; receiving, by the computer subsystem, a position of a guide tube in navigational space; determining, by the computer, a distance between a distal portion of the guide tube to the target bone of the patient, wherein the distance is determined such that a central axis of the guide tube is coincident to the intersection; and depicting, via the display, a vector indicative of the distance to contact the target bone of the patient on at least one of the first viewplane scan and the second viewplane scan and further comprising an end-effector wherein the guide tube is part of the end effector.

According to another unclaimed aspect it is provided a method for determining the distance for a surgical instrument to contact a target bone of a patient during a surgical operating using a robotic surgical system, said method comprising: receiving, by a computer subsystem having a computer and a display, a first viewplane scan of the target bone and a second viewplane scan of the target bone, wherein the first viewplane and the second viewplane form an intersection of views of the target bone in an image space; receiving, by the computer subsystem, a position of a guide tube in navigational space; determining, by the computer, a distance between a distal portion of the guide tube to the target bone of the patient, wherein the distance is determined such that a central axis of the guide tube is coincident to the intersection; and depicting, via the display, a vector indicative of the distance to contact the target bone of the patient on at least one of the first viewplane scan and the second viewplane scan and further comprising an end-effector and wherein the guide tube is tracked separately from the end effector.

### BRIEF DESCRIPTION OF THE DRAWINGS

The invention and the following detailed description of certain embodiments thereof may be understood by reference to the following figures:
FIG. 1 is an overhead view of a potential arrangement for locations of the robotic system, patient, surgeon, and other medical personnel during a surgical procedure;
FIG. 2 illustrates the robotic system including positioning of the surgical robot and the camera relative to the patient according to one embodiment;
FIG. 3 illustrates a surgical robotic system in accordance with an exemplary embodiment;
FIG. 4 illustrates a portion of a surgical robot in accordance with an exemplary embodiment;
FIG. 5 illustrates a block diagram of a surgical robot in accordance with an exemplary embodiment;
FIG. 6 illustrates a surgical robot in accordance with an exemplary embodiment;
FIGS. 7A-7C illustrate an end effector in accordance with an exemplary embodiment;
FIG. 8 illustrates a surgical instrument and the end effector, before and after, inserting the surgical instrument into the guide tube of the end effector according to one embodiment;
FIGS. 9A-9C illustrate portions of an end effector and robot arm in accordance with an exemplary embodiment;
FIG. 10 illustrates a dynamic reference array, an imaging array, and other components in accordance with an exemplary embodiment;
FIG. 11 illustrates a method of registration in accordance with an exemplary embodiment;
FIG. 12A-12B illustrate embodiments of imaging devices according to exemplary embodiments;
FIG. 13 illustrates an exemplary image of a viewplane x-ray with associated depictions measuring depth to a target bone according to the invention;
FIG. 14 illustrates an exemplary image of a viewplane of an x-ray with associated depictions measuring depth to a target bone according to the invention;
FIG. 15 illustrates an exemplary image of a viewplane of an x-ray with associated depictions measuring depth to a target bone according to the invention;
FIG. 16 illustrates an exemplary image of a viewplane of an x-ray with associated depictions measuring depth to a target bone according to an exemplary embodiment of the invention;
FIG. 17 illustrates an exemplary image of a viewplane of an x-ray with associated depictions measuring depth to a target bone according to an exemplary embodiment of the invention;
FIG. 18 illustrates an exemplary instrument with markings according to an exemplary embodiment of the invention;
FIG. 19 illustrates an exemplary instrument and guide tube according to an exemplary embodiment of the invention;
FIG. 20 illustrates an exemplary method consistent with the present disclosure.

### DETAILED DESCRIPTION OF THE INVENTION

It is to be understood that the present disclosure is not limited in its application to the details of construction and the arrangement of components set forth in the description herein or illustrated in the drawings. The teachings of the present disclosure may be used and practiced in other embodiments and practiced or carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Unless specified or limited otherwise, the terms "mounted," "connected," "supported," and "coupled" and variations thereof are used broadly and encompass both direct and indirect mountings, connections, supports, and couplings. Further, "connected" and "coupled" are not restricted to physical or mechanical connections or couplings.

The following discussion is presented to enable a person skilled in the art to make and use embodiments of the present disclosure. Various modifications to the illustrated embodiments will be readily apparent to those skilled in the art, and the principles herein can be applied to other embodiments and applications without departing from embodiments of the present disclosure. Thus, the embodiments are not intended to be limited to embodiments shown, but are to be accorded the widest scope consistent with the principles and features disclosed herein. The following detailed description is to be read with reference to the figures, in which like elements in different figures have like reference numerals. The figures, which are not necessarily to scale, depict selected embodiments and are not intended to limit the scope of the embodiments. Skilled artisans will recognize the examples provided herein have many useful alternatives and fall within the scope of the embodiments.

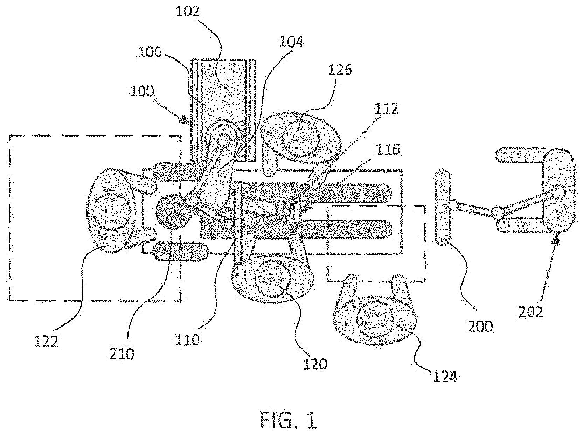

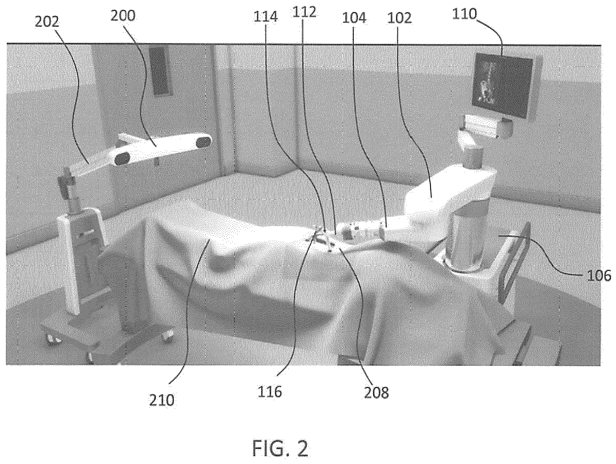

Turning now to the drawing, FIGS. 1 and 2 illustrate a surgical robot system 100 in accordance with an exemplary embodiment. Surgical robot system 100 may include, for example, a surgical robot 102, one or more robot arms 104, a base 106, a display 110, an end effector 112, for example, including a guide tube 114, and one or more tracking markers 118. The surgical robot system 100 may include a patient tracking device 116 also including one or more tracking markers 118, which is adapted to be secured directly to the patient 210 (e.g., to the bone of the patient 210). The surgical robot system 100 may also utilize a camera 200, for example, positioned on a camera stand 202. The camera stand 202 can have any suitable configuration to move, orient, and support the camera 200 in a desired position. The camera 200 may include any suitable camera or cameras, such as one or more infrared cameras (e.g., bifocal or stereophotogrammetric cameras), able to identify, for example, active and passive tracking markers 118 in a given measurement volume viewable from the perspective of the camera 200. The camera 200 may scan the given measurement volume and detect the light that comes from the markers 118 in order to identify and determine the position of the markers 118 in three dimensions. For example, active markers 118 may include infrared-emitting markers that are activated by an electrical signal (e.g., infrared light emitting diodes (LEDs)), and passive markers 118 may include retro-reflective markers that reflect infrared light (e.g., they reflect incoming IR radiation into the direction of the incoming light), for example, emitted by illuminators on the camera 200 or other suitable device.

FIGS. 1 and 2 illustrate a potential configuration for the placement of the surgical robot system 100 in an operating room environment. For example, the robot 102 may be positioned near or next to patient 210. Although depicted near the head of the patient 210, it will be appreciated that the robot 102 can be positioned at any suitable location near the patient 210 depending on the area of the patient 210 undergoing the operation. The camera 200 may be separated from the robot system 100 and positioned at the foot of patient 210. This location allows the camera 200 to have a direct visual line of sight to the surgical field 208. Again, it is contemplated that the camera 200 may be located at any suitable position having line of sight to the surgical field 208. In the configuration shown, the surgeon 120 may be positioned across from the robot 102, but is still able to manipulate the end effector 112 and the display 110. A surgical assistant 126 may be positioned across from the surgeon 120 again with access to both the end effector 112 and the display 110. If desired, the locations of the surgeon 120 and the assistant 126 may be reversed. The traditional areas for the anesthesiologist 122 and the nurse or scrub tech 124 remain unimpeded by the locations of the robot 102 and camera 200.

With respect to the other components of the robot 102, the display 110 can be attached to the surgical robot 102 and in other exemplary embodiments, display 110 can be detached from surgical robot 102, either within a surgical room with the surgical robot 102, or in a remote location. End effector 112 may be coupled to the robot arm 104 and controlled by at least one motor. In exemplary embodiments, end effector 112 can comprise a guide tube 114, which is able to receive and orient a surgical instrument 608 (described further herein) used to perform surgery on the patient 210. As used herein, the term "end effector" is used interchangeably with the terms "end-effectuator" and "effectuator element." Although generally shown with a guide tube 114, it will be appreciated that the end effector 112 may be replaced with any suitable instrumentation suitable for use in surgery. In some embodiments, end effector 112 can comprise any known structure for effecting the movement of the surgical instrument 608 in a desired manner.

The surgical robot 102 is able to control the translation and orientation of the end effector 112. The robot 102 is able to move end effector 112 along x-, y-, and z-axes, for example. The end effector 112 can be configured for selective rotation about one or more of the x-, y-, and z- axis, and a Z Frame axis (such that one or more of the Euler Angles (e.g., roll, pitch, and/or yaw) associated with end effector 112 can be selectively controlled). In some exemplary embodiments, selective control of the translation and orientation of end effector 112 can permit performance of medical procedures with significantly improved accuracy compared to conventional robots that utilize, for example, a six degree of freedom robot arm comprising only rotational axes. For example, the surgical robot system 100 may be used to operate on patient 210, and robot arm 104 can be positioned above the body of patient 210, with end effector 112 selectively angled relative to the z-axis toward the body of patient 210.

In some exemplary embodiments, the position of the surgical instrument 608 can be dynamically updated so that surgical robot 102 can be aware of the location of the surgical instrument 608 at all times during the procedure. Consequently, in some exemplary embodiments, surgical robot 102 can move the surgical instrument 608 to the desired position quickly without any further assistance from a physician (unless the physician so desires). In some further embodiments, surgical robot 102 can be configured to correct the path of the surgical instrument 608 if the surgical instrument 608 strays from the selected, preplanned trajectory. In some exemplary embodiments, surgical robot 102 can be configured to permit stoppage, modification, and/or manual control of the movement of end effector 112 and/or the surgical instrument 608. Thus, in use, in exemplary embodiments, a physician or other user can operate the system 100, and has the option to stop, modify, or manually control the autonomous movement of end effector 112 and/or the surgical instrument 608. Further details of surgical robot system 100 including the control and movement of a surgical instrument 608 by surgical robot 102 can be found in co-pending U.S. patent application Ser. No. 13/924,505, published as US 2013-0345718 A1.

The robotic surgical system 100 can comprise one or more tracking markers 118 configured to track the movement of robot arm 104, end effector 112, patient 210, and/or the surgical instrument 608 in three dimensions. In exemplary embodiments, a plurality of tracking markers 118 can be mounted (or otherwise secured) thereon to an outer surface of the robot 102, such as, for example and without limitation, on base 106 of robot 102, on robot arm 104, or on the end effector 112. In exemplary embodiments, at least one tracking marker 118 of the plurality of tracking markers 118 can be mounted or otherwise secured to the end effector 112. One or more tracking markers 118 can further be mounted (or otherwise secured) to the patient 210. In exemplary embodiments, the plurality of tracking markers 118 can be positioned on the patient 210 spaced apart from the surgical field 208 to reduce the likelihood of being obscured by the surgeon, surgical tools, or other parts of the robot 102. Further, one or more tracking markers 118 can be further mounted (or otherwise secured) to the surgical tools 608 (e.g., a screw driver, dilator, implant inserter, or the like). Thus, the tracking markers 118 enable each of the marked objects (e.g., the end effector 112, the patient 210, and the surgical tools 608) to be tracked by the robot 102. In exemplary embodiments, system 100 can use tracking information collected from each of the marked objects to calculate the orientation and location, for example, of the end effector 112, the surgical instrument 608 (e.g., positioned in the tube 114 of the end effector 112), and the relative position of the patient 210.

In exemplary embodiments, one or more of markers 118 may be optical markers. In some embodiments, the positioning of one or more tracking markers 118 on end effector 112 can maximize the accuracy of the positional measurements by serving to check or verify the position of end effector 112. Further details of surgical robot system 100 including the control, movement and tracking of surgical robot 102 and of a surgical instrument 608 can be found in co-pending U.S. patent application Ser. No. 13/924,505, published as US 2013-0345718 A1.

Exemplary embodiments include one or more markers 118 coupled to the surgical instrument 608. In exemplary embodiments, these markers 118, for example, coupled to the patient 210 and surgical instruments 608, as well as markers 118 coupled to the end effector 112 of the robot 102 can comprise conventional infrared light-emitting diodes (LEDs) or an Optotrak^{®} diode capable of being tracked using a commercially available infrared optical tracking system such as Optotrak^{®}. Optotrak^{®} is a registered trademark of Northern Digital Inc., Waterloo, Ontario, Canada. In other embodiments, markers 118 can comprise conventional reflective spheres capable of being tracked using a commercially available optical tracking system such as Polaris Spectra. Polaris Spectra is also a registered trademark of Northern Digital, Inc. In an exemplary embodiment, the markers 118 coupled to the end effector 112 are active markers which comprise infrared light-emitting diodes which may be turned on and off, and the markers 118 coupled to the patient 210 and the surgical instruments 608 comprise passive reflective spheres.

In exemplary embodiments, light emitted from and/or reflected by markers 118 can be detected by camera 200 and can be used to monitor the location and movement of the marked objects. In alternative embodiments, markers 118 can comprise a radio-frequency and/or electromagnetic reflector or transceiver and the camera 200 can include or be replaced by a radio-frequency and/or electromagnetic transceiver.

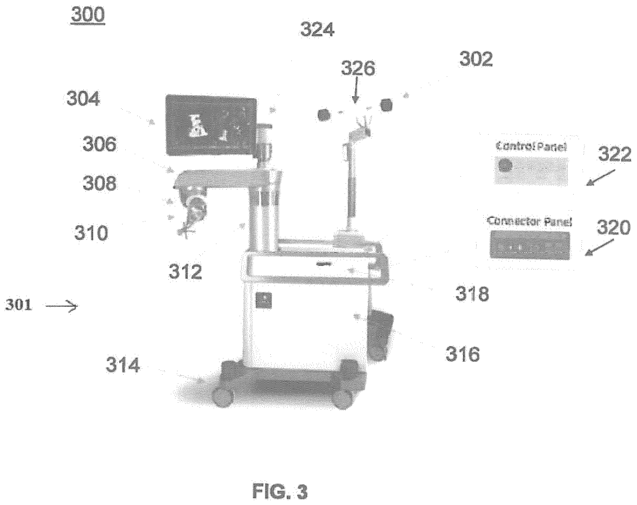

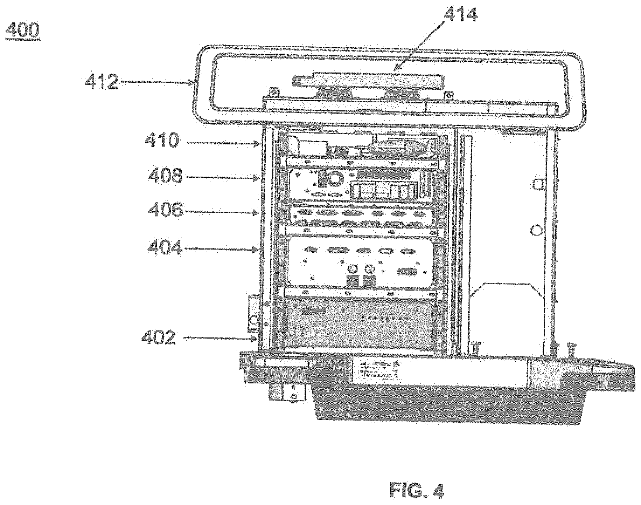

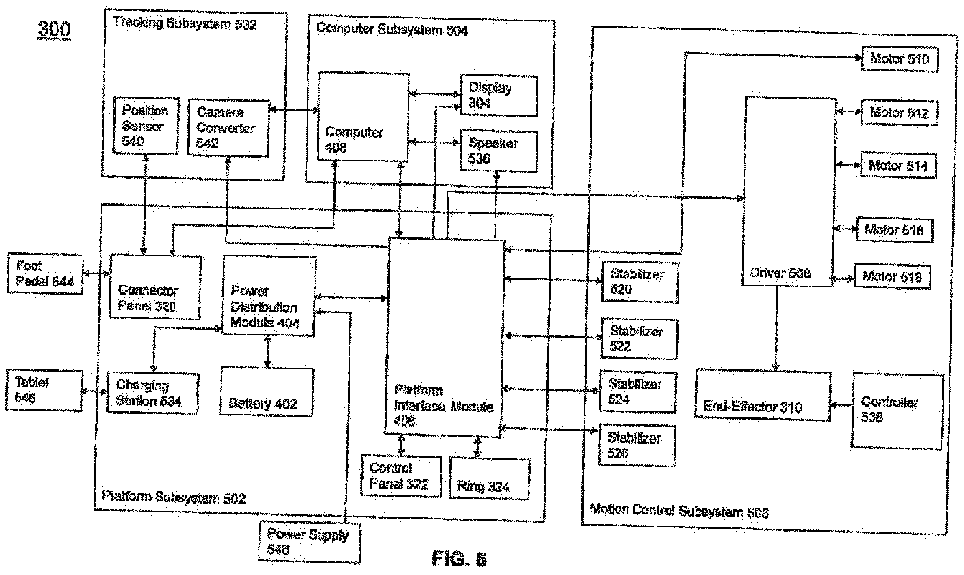

Similar to surgical robot system 100, FIG. 3 illustrates a surgical robot system 300 and camera stand 302, in a docked configuration, consistent with an exemplary embodiment of the present disclosure. Surgical robot system 300 may comprise a robot 301 including a display 304, upper arm 306, lower arm 308, end effector 310, vertical column 312, casters 314, cabinet 316, tablet drawer 318, connector panel 320, control panel 322, and ring of information 324. Camera stand 302 may comprise camera 326. These components are described in greater with respect to FIG. 5. FIG. 3 illustrates the surgical robot system 300 in a docked configuration where the camera stand 302 is nested with the robot 301, for example, when not in use. It will be appreciated by those skilled in the art that the camera 326 and robot 301 may be separated from one another and positioned at any appropriate location during the surgical procedure, for example, as shown in FIGS. 1 and 2. FIG. 4 illustrates a base 400 consistent with an exemplary embodiment of the present disclosure. Base 400 may be a portion of surgical robot system 300 and comprise cabinet 316. Cabinet 316 may house certain components of surgical robot system 300 including but not limited to a battery 402, a power distribution module 404, a platform interface board module 406, a computer 408, a handle 412, and a tablet drawer 414. The connections and relationship between these components is described in greater detail with respect to FIG. 5.

FIG. 5 illustrates a block diagram of certain components of an exemplary embodiment of surgical robot system 300. Surgical robot system 300 may comprise platform subsystem 502, computer subsystem 504, motion control subsystem 506, and tracking subsystem 532. Platform subsystem 502 may further comprise battery 402, power distribution module 404, platform interface board module 406, and tablet charging station 534. Computer subsystem 504 may further comprise computer 408, display 304, and speaker 536. Motion control subsystem 506 may further comprise driver circuit 508, motors 510, 512, 514, 516, 518, stabilizers 520, 522, 524, 526, end effector 310, and controller 538. Tracking subsystem 532 may further comprise position sensor 540 and camera converter 542. System 300 may also comprise a foot pedal 544 and tablet 546.

Input power is supplied to system 300 via a power source 548 which may be provided to power distribution module 404. Power distribution module 404 receives input power and is configured to generate different power supply voltages that are provided to other modules, components, and subsystems of system 300. Power distribution module 404 may be configured to provide different voltage supplies to platform interface module 406, which may be provided to other components such as computer 408, display 304, speaker 536, driver 508 to, for example, power motors 512, 514, 516, 518 and end effector 310, motor 510, ring 324, camera converter 542, and other components for system 300 for example, fans for cooling the electrical components within cabinet 316.

Power distribution module 404 may also provide power to other components such as tablet charging station 534 that may be located within tablet drawer 318. Tablet charging station 534 may be in wireless or wired communication with tablet 546 for charging table 546. Tablet 546 may be used by a surgeon consistent with the present disclosure and described herein. Power distribution module 404 may also be connected to battery 402, which serves as temporary power source in the event that power distribution module 404 does not receive power from input power 548. At other times, power distribution module 404 may serve to charge battery 402 if necessary.

Other components of platform subsystem 502 may also include connector panel 320, control panel 322, and ring 324. Connector panel 320 may serve to connect different devices and components to system 300 and/or associated components and modules. Connector panel 320 may contain one or more ports that receive lines or connections from different components. For example, connector panel 320 may have a ground terminal port that may ground system 300 to other equipment, a port to connect foot pedal 544 to system 300, a port to connect to tracking subsystem 532, which may comprise position sensor 540, camera converter 542, and cameras 326 associated with camera stand 302. Connector panel 320 may also include other ports to allow USB, Ethernet, HDMI communications to other components, such as computer 408.

Control panel 322 may provide various buttons or indicators that control operation of system 300 and/or provide information regarding system 300. For example, control panel 322 may include buttons to power on or off system 300, lift or lower vertical column 312, and lift or lower stabilizers 520-526 that may be designed to engage casters 314 to lock system 300 from physically moving. Other buttons may stop system 300 in the event of an emergency, which may remove all motor power and apply mechanical brakes to stop all motion from occurring. Control panel 322 may also have indicators notifying the user of certain system conditions such as a line power indicator or status of charge for battery 402.

Ring 324 may be a visual indicator to notify the user of system 300 of different modes that system 300 is operating under and certain warnings to the user.

Computer subsystem 504 includes computer 408, display 304, and speaker 536. Computer 504 includes an operating system and software to operate system 300. Computer 504 may receive and process information from other components (for example, tracking subsystem 532, platform subsystem 502, and/or motion control subsystem 506) in order to display information to the user. Further, computer subsystem 504 may also include speaker 536 to provide audio to the user.

Tracking subsystem 532 may include position sensor 504 and converter 542. Tracking subsystem 532 may correspond to camera stand 302 including camera 326 as described with respect to FIG. 3. Position sensor 504 may be camera 326. Tracking subsystem may track the location of certain markers that are located on the different components of system 300 and/or instruments used by a user during a surgical procedure. This tracking may be conducted in a manner consistent with the present disclosure including the use of infrared technology that tracks the location of active or passive elements, such as LEDs or reflective markers, respectively. The location, orientation, and position of structures having these types of markers may be provided to computer 408 which may be shown to a user on display 304. For example, a surgical instrument 608 having these types of markers and tracked in this manner (which may be referred to as a navigational space) may be shown to a user in relation to a three dimensional image of a patient's anatomical structure. Motion control subsystem 506 may be configured to physically move vertical column 312, upper arm 306, lower arm 308, or rotate end effector 310. The physical movement may be conducted through the use of one or more motors 510-518. For example, motor 510 may be configured to vertically lift or lower vertical column 312. Motor 512 may be configured to laterally move upper arm 308 around a point of engagement with vertical column 312 as shown in FIG. 3. Motor 514 may be configured to laterally move lower arm 308 around a point of engagement with upper arm 308 as shown in FIG. 3. Motors 516 and 518 may be configured to move end effector 310 in a manner such that one may control the roll and one may control the tilt, thereby providing multiple angles that end effector 310 may be moved. These movements may be achieved by controller 538 which may control these movements through load cells disposed on end effector 310 and activated by a user engaging these load cells to move system 300 in a desired manner.

Moreover, system 300 may provide for automatic movement of vertical column 312, upper arm 306, and lower arm 308 through a user indicating on display 304 (which may be a touchscreen input device) the location of a surgical instrument or component on three dimensional image of the patient's anatomy on display 304. The user may initiate this automatic movement by stepping on foot pedal 544 or some other input means.

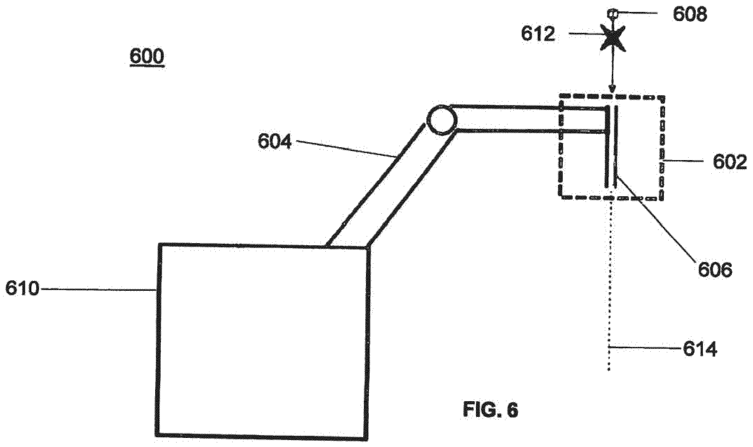

FIG. 6 illustrates a surgical robot system 600 consistent with an exemplary embodiment. Surgical robot system 600 may comprise end effector 602, robot arm 604, guide tube 606, instrument 608, and robot base 610. Instrument tool 608 may be attached to a tracking array 612 including one or more tracking markers (such as markers 118) and have an associated trajectory 614. Trajectory 614 may represent a path of movement that instrument tool 608 is configured to travel once it is positioned through or secured in guide tube 606, for example, a path of insertion of instrument tool 608 into a patient. In an exemplary operation, robot base 610 may be configured to be in electronic communication with robot arm 604 and end effector 602 so that surgical robot system 600 may assist a user (for example, a surgeon) in operating on the patient 210. Surgical robot system 600 may be consistent with previously described surgical robot system 100 and 300.

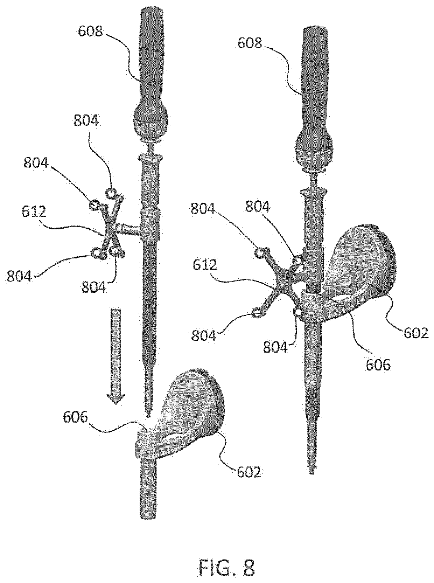

A tracking array 612 may be mounted on instrument 608 to monitor the location and orientation of instrument tool 608. The tracking array 612 may be attached to an instrument 608 and may comprise tracking markers 804. As best seen in FIG. 8, tracking markers 804 may be, for example, light emitting diodes and/or other types of reflective markers (e.g., markers 118 as described elsewhere herein). The tracking devices may be one or more line of sight devices associated with the surgical robot system. As an example, the tracking devices may be one or more cameras 200, 326 associated with the surgical robot system 100, 300 and may also track tracking array 612 for a defined domain or relative orientations of the instrument 608 in relation to the robot arm 604, the robot base 610, end effector 602, and/or the patient 210. The tracking devices may be consistent with those structures described in connection with camera stand 302 and tracking subsystem 532.

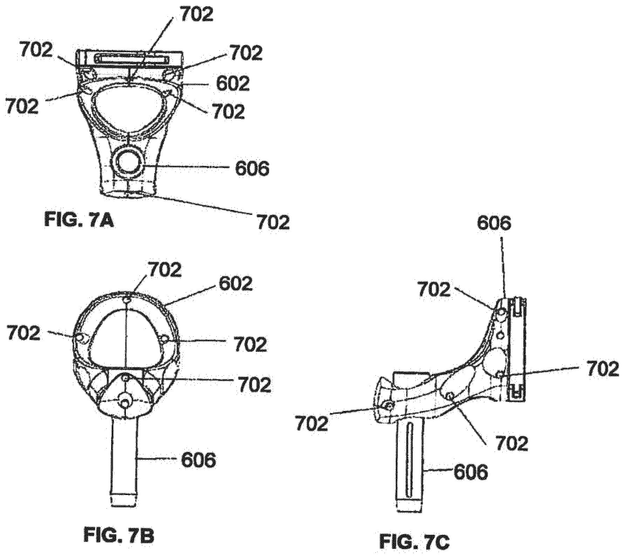

FIGS. 7A, 7B, and 7C illustrate a top view, front view, and side view, respectively, of end effector 602 consistent with an exemplary embodiment. End effector 602 may comprise one or more tracking markers 702. Tracking markers 702 may be light emitting diodes or other types of active and passive markers, such as tracking markers 118 that have been previously described. In an exemplary embodiment, the tracking markers 702 are active infrared-emitting markers that are activated by an electrical signal (e.g., infrared light emitting diodes (LEDs)). Thus, tracking markers 702 may be activated such that the infrared markers 702 are visible to the camera 200, 326 or may be deactivated such that the infrared markers 702 are not visible to the camera 200, 326. Thus, when the markers 702 are active, the end effector 602 may be controlled by the system 100, 300, 600, and when the markers 702 are deactivated, the end effector 602 may be locked in position and unable to be moved by the system 100, 300, 600.

Markers 702 may be disposed on or within end effector 602 in a manner such that the markers 702 are visible by one or more cameras 200, 326 or other tracking devices associated with the surgical robot system 100, 300, 600. The camera 200, 326 or other tracking devices may track end effector 602 as it moves to different positions and viewing angles by following the movement of tracking markers 702. The location of markers 702 and/or end effector 602 may be shown on a display 110, 304 associated with the surgical robot system 100, 300, 600, for example, display 110 as shown in FIG. 2 and/or display 304 shown in FIG. 3. This display 110, 304 may allow a user to ensure that end effector 602 is in a desirable position in relation to robot arm 604, robot base 610, the patient 210, and/or the user.

For example, as shown in FIG. 7A, according to the invention, markers 720 are placed around the surface of end effector 602 so that a tracking device placed away from the surgical field 208 and facing toward the robot 102, 301 and the camera 200, 326 is able to view at least 3 of the markers 702 through a range of common orientations of the end effector 602 relative to the tracking device 100, 300, 600. For example, distribution of markers 702 in this way allows end effector 602 to be monitored by the tracking devices when end effector 602 is translated and rotated in the surgical field 208.

In addition, in exemplary embodiments, end effector 602 may be equipped with infrared (IR) receivers that can detect when an external camera 200, 326 is getting ready to read markers 702. Upon this detection, end effector 602 may then illuminate markers 702. The detection by the IR receivers that the external camera200, 326 is ready to read markers 702 may signal the need to synchronize a duty cycle of markers 702, which may be light emitting diodes, to an external camera200, 326. This may also allow for lower power consumption by the robotic system as a whole, whereby markers 702 would only be illuminated at the appropriate time instead of being illuminated continuously. Further, in exemplary embodiments, markers 702 may be powered off to prevent interference with other navigation tools, such as different types of surgical instruments 608.

FIG. 8 depicts one type of surgical instrument 608 including a tracking array 612 and tracking markers 804. Tracking markers 804 may be of any type described herein including but not limited to light emitting diodes or reflective spheres. Markers 804 are monitored by tracking devices associated with the surgical robot system 100, 300, 600 and may be one or more of the line of sight cameras 200, 326. The cameras 200, 326 may track the location of instrument 608 based on the position and orientation of tracking array 612 and markers 804. A user, such as a surgeon 120, may orient instrument 608 in a manner so that tracking array 612 and markers 804 are sufficiently recognized by the tracking device or camera 200, 326 to display instrument 608 and markers 804 on, for example, display 110 of the exemplary surgical robot system.

The manner in which a surgeon 120 may place instrument 608 into guide tube 606 of the end effector 602 and adjust the instrument 608 is evident in FIG. 8. The hollow tube or guide tube 114, 606 of the end effector 112, 310, 602 is sized and configured to receive at least a portion of the surgical instrument 608. The guide tube 114, 606 is configured to be oriented by the robot arm 104 such that insertion and trajectory for the surgical instrument 608 is able to reach a desired anatomical target within or upon the body of the patient 210. The surgical instrument 608 may include at least a portion of a generally cylindrical instrument. Although a screw driver is exemplified as the surgical tool 608, it will be appreciated that any suitable surgical tool 608 may be positioned by the end effector 602. By way of example, the surgical instrument 608 may include one or more of a guide wire, cannula, a retractor, a drill, a reamer, a screw driver, an insertion tool, a removal tool, or the like. Although the hollow tube 114, 606 is generally shown as having a cylindrical configuration, it will be appreciated by those of skill in the art that the guide tube 114, 606 may have any suitable shape, size and configuration desired to accommodate the surgical instrument 608 and access the surgical site.

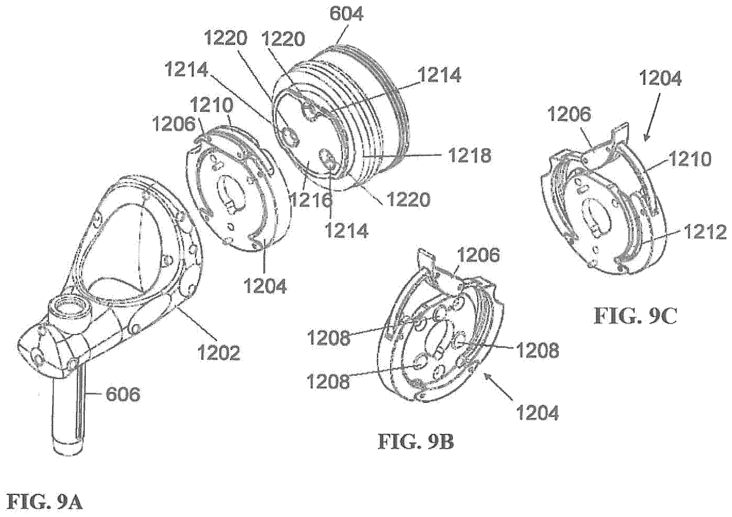

FIGS. 9A-9C illustrate end effector 602 and a portion of robot arm 604 consistent with an exemplary embodiment. End effector 602 may further comprise body 1202 and clamp 1204. Clamp 1204 may comprise handle 1206, balls 1208, spring 1210, and lip 1212. Robot arm 604 may further comprise depressions 1214, mounting plate 1216, lip 1218, and magnets 1220.

End effector 602 may mechanically interface and/or engage with the surgical robot system and robot arm 604 through one or more couplings. For example, end effector 602 may engage with robot arm 604 through a locating coupling and/or a reinforcing coupling. Through these couplings, end effector 602 may fasten with robot arm 604 outside a flexible and sterile barrier. In an exemplary embodiment, the locating coupling may be a magnetically kinematic mount and the reinforcing coupling may be a five bar over center clamping linkage.

With respect to the locating coupling, robot arm 604 may comprise mounting plate 1216, which may be non-magnetic material, one or more depressions 1214, lip 1218, and magnets 1220. Magnet 1220 is mounted below each of depressions 1214. Portions of clamp 1204 may comprise magnetic material and be attracted by one or more magnets 1220. Through the magnetic attraction of clamp 1204 and robot arm 604, balls 1208 become seated into respective depressions 1214. For example, balls 1208 as shown in FIG. 9B would be seated in depressions 1214 as shown in FIG. 9A. This seating may be considered a magnetically-assisted kinematic coupling. Magnets 1220 may be configured to be strong enough to support the entire weight of end effector 602 regardless of the orientation of end effector 602. The locating coupling may be any style of kinematic mount that uniquely restrains six degrees of freedom.

With respect to the reinforcing coupling, portions of clamp 1204 may be configured to be a fixed ground link and as such clamp 1204 may serve as a five bar linkage. Closing clamp handle 1206 may fasten end effector 602 to robot arm 604 as lip 1212 and lip 1218 engage clamp 1204 in a manner to secure end effector 602 and robot arm 604. When clamp handle 1206 is closed, spring 1210 may be stretched or stressed while clamp 1204 is in a locked position. The locked position may be a position that provides for linkage past center. Because of a closed position that is past center, the linkage will not open absent a force applied to clamp handle 1206 to release clamp 1204. Thus, in a locked position end effector 602 may be robustly secured to robot arm 604.

Spring 1210 may be a curved beam in tension. Spring 1210 may be comprised of a material that exhibits high stiffness and high yield strain such as virgin PEEK (poly-ether-etherketone). The linkage between end effector 602 and robot arm 604 may provide for a sterile barrier between end effector 602 and robot arm 604 without impeding fastening of the two couplings.

The reinforcing coupling may be a linkage with multiple spring members. The reinforcing coupling may latch with a cam or friction based mechanism. The reinforcing coupling may also be a sufficiently powerful electromagnet that will support fastening end- effector 102 to robot arm 604. The reinforcing coupling may be a multi-piece collar completely separate from either end effector 602 and/or robot arm 604 that slips over an interface between end effector 602 and robot arm 604 and tightens with a screw mechanism, an over center linkage, or a cam mechanism.

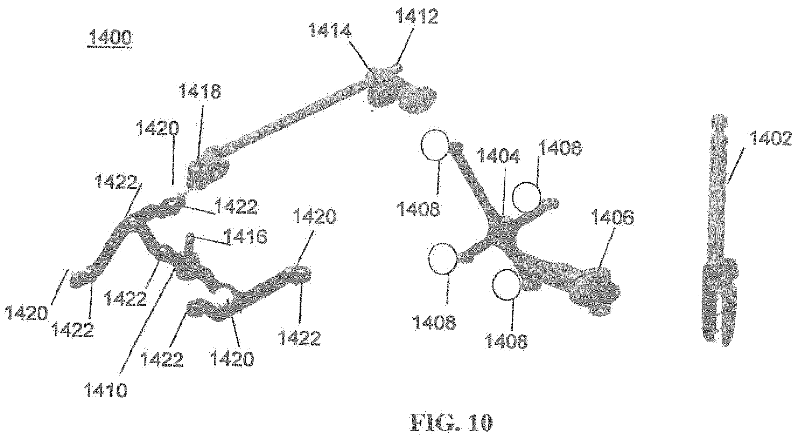

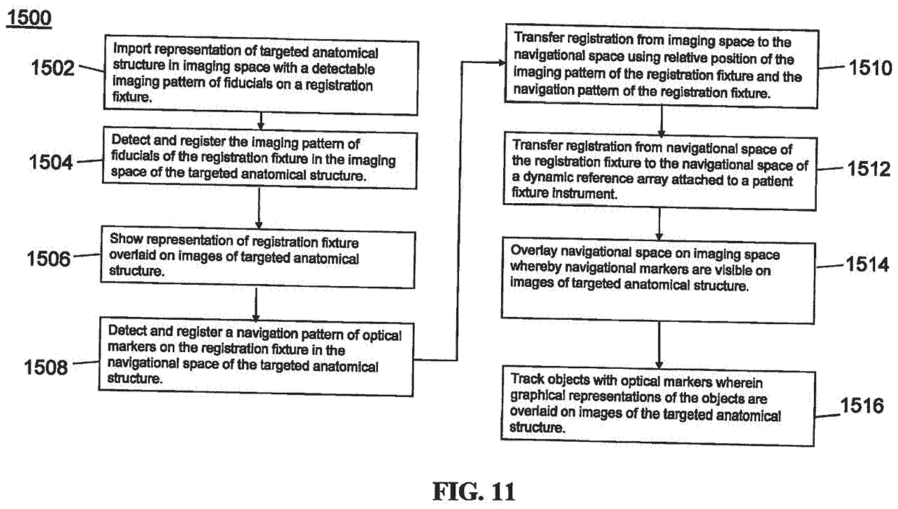

Referring to FIGS. 10 and 11, prior to or during a surgical procedure, certain registration procedures may be conducted in order to track objects and a target anatomical structure of the patient 210 both in a navigation space and an image space. In order to conduct such registration, according to the invention, a registration system 1400 is used as illustrated in FIG. 10.

In order to track the position of the patient 210, a patient tracking device 116 may include a patient fixation instrument 1402 to be secured to a rigid anatomical structure of the patient 210 and a dynamic reference base (DRB) 1404 may be securely attached to the patient fixation instrument 1402. For example, patient fixation instrument 1402 may be inserted into opening 1406 of dynamic reference base 1404. Dynamic reference base 1404 may contain markers 1408 that are visible to tracking devices, such as tracking subsystem 532. These markers 1408 may be optical markers or reflective spheres, such as tracking markers 118, as previously discussed herein.

Patient fixation instrument 1402 is attached to a rigid anatomy of the patient 210 and may remain attached throughout the surgical procedure. In an exemplary embodiment, patient fixation instrument 1402 is attached to a rigid area of the patient 210, for example, a bone that is located away from the targeted anatomical structure subject to the surgical procedure. In order to track the targeted anatomical structure, dynamic reference base 1404 is associated with the targeted anatomical structure through the use of a registration fixture that is temporarily placed on or near the targeted anatomical structure in order to register the dynamic reference base 1404 with the location of the targeted anatomical structure.

A registration fixture 1410 is attached to patient fixation instrument 1402 through the use of a pivot arm 1412. Pivot arm 1412 is attached to patient fixation instrument 1402 by inserting patient fixation instrument 1402 through an opening 1414 of registration fixture 1410. Pivot arm 1412 is attached to registration fixture 1410 by, for example, inserting a knob 1416 through an opening 1418 of pivot arm 1412.

Using pivot arm 1412, registration fixture 1410 may be placed over the targeted anatomical structure and its location may be determined in an image space and navigation space using tracking markers 1420 and/or fiducials 1422 on registration fixture 1410. Registration fixture 1410 may contain a collection of markers 1420 that are visible in a navigational space (for example, markers 1420 may be detectable by tracking subsystem 532). Tracking markers 1420 may be optical markers visible in infrared light as previously described herein. Registration fixture 1410 may also contain a collection of fiducials 1422, for example, such as bearing balls, that are visible in an imaging space (for example, a three dimension CT image). As described in greater detail with respect to FIG. 11, using registration fixture 1410, the targeted anatomical structure may be associated with dynamic reference base 1404 thereby allowing depictions of objects in the navigational space to be overlaid on images of the anatomical structure. Dynamic reference base 1404, located at a position away from the targeted anatomical structure, may become a reference point thereby allowing removal of registration fixture 1410 and/or pivot arm 1412 from the surgical area.

FIG. 11 provides an exemplary method 1500 for registration consistent with the present disclosure. Method 1500 begins at step 1502 wherein a graphical representation (or image(s)) of the targeted anatomical structure may be imported into system 100, 300 600, for example computer 408. The graphical representation may be three dimensional CT or a fluoroscope scan of the targeted anatomical structure of the patient 210 which includes registration fixture 1410 and a detectable imaging pattern of fiducials 1420.

At step 1504, an imaging pattern of fiducials 1420 is detected and registered in the imaging space and stored in computer 408. Optionally, at this time at step 1506, a graphical representation of the registration fixture 1410 may be overlaid on the images of the targeted anatomical structure.

At step 1508, a navigational pattern of registration fixture 1410 is detected and registered by recognizing markers 1420. Markers 1420 may be optical markers that are recognized in the navigation space through infrared light by tracking subsystem 532 via position sensor 540. Thus, the location, orientation, and other information of the targeted anatomical structure is registered in the navigation space. Therefore, registration fixture 1410 may be recognized in both the image space through the use of fiducials 1422 and the navigation space through the use of markers 1420. At step 1510, the registration of registration fixture 1410 in the image space is transferred to the navigation space. This transferal is done, for example, by using the relative position of the imaging pattern of fiducials 1422 compared to the position of the navigation pattern of markers 1420.

At step 1512, registration of the navigation space of registration fixture 1410 (having been registered with the image space) is further transferred to the navigation space of dynamic registration array 1404 attached to patient fixture instrument 1402. Thus, registration fixture 1410 may be removed and dynamic reference base 1404 may be used to track the targeted anatomical structure in both the navigation and image space because the navigation space is associated with the image space.

At steps 1514 and 1516, the navigation space may be overlaid on the image space and objects with markers visible in the navigation space (for example, surgical instruments 608 with optical markers 804). The objects may be tracked through graphical representations of the surgical instrument 608 on the images of the targeted anatomical structure.

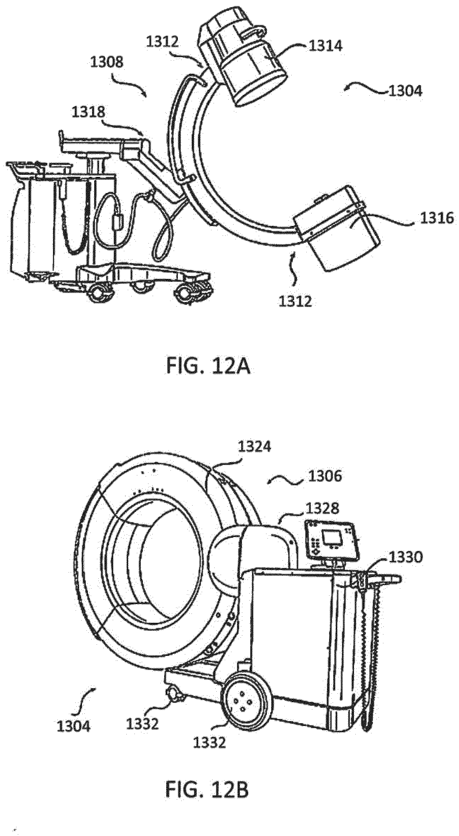

FIGS. 12A-12B illustrate imaging devices 1304 that may be used in conjunction with robot systems 100, 300, 600 to acquire pre-operative, intra-operative, post-operative, and/or real- time image data of patient 210. Any appropriate subject matter may be imaged for any appropriate procedure using the imaging system 1304. The imaging system 1304 may be any imaging device such as imaging device 1306 and/or a C-arm 1308 device. It may be desirable to take x-rays of patient 210 from a number of different positions, without the need for frequent manual repositioning of patient 210 which may be required in an x-ray system. As illustrated in Figure 12A, the imaging system 1304 may be in the form of a C-arm 1308 that includes an elongated C-shaped member terminating in opposing distal ends 1312 of the "C" shape. C-shaped member 1130 may further comprise an x-ray source 1314 and an image receptor 1316. The space within C-arm 1308 of the arm may provide room for the physician to attend to the patient substantially free of interference from x-ray support structure 1318. As illustrated in FIG. 12B, the imaging system may include imaging device 1306 having a gantry housing 1324 attached to a support structure imaging device support structure 1328, such as a wheeled mobile cart 1330 with wheels 1332, which may enclose an image capturing portion, not illustrated. The image capturing portion may include an x-ray source and/or emission portion and an x-ray receiving and/or image receiving portion, which may be disposed about one hundred and eighty degrees from each other and mounted on a rotor (not illustrated) relative to a track of the image capturing portion. The image capturing portion may be operable to rotate three hundred and sixty degrees during image acquisition. The image capturing portion may rotate around a central point and/or axis, allowing image data of patient 210 to be acquired from multiple directions or in multiple planes. Although certain imaging systems 1304 are exemplified herein, it will be appreciated that any suitable imaging system may be selected by one of ordinary skill in the art.

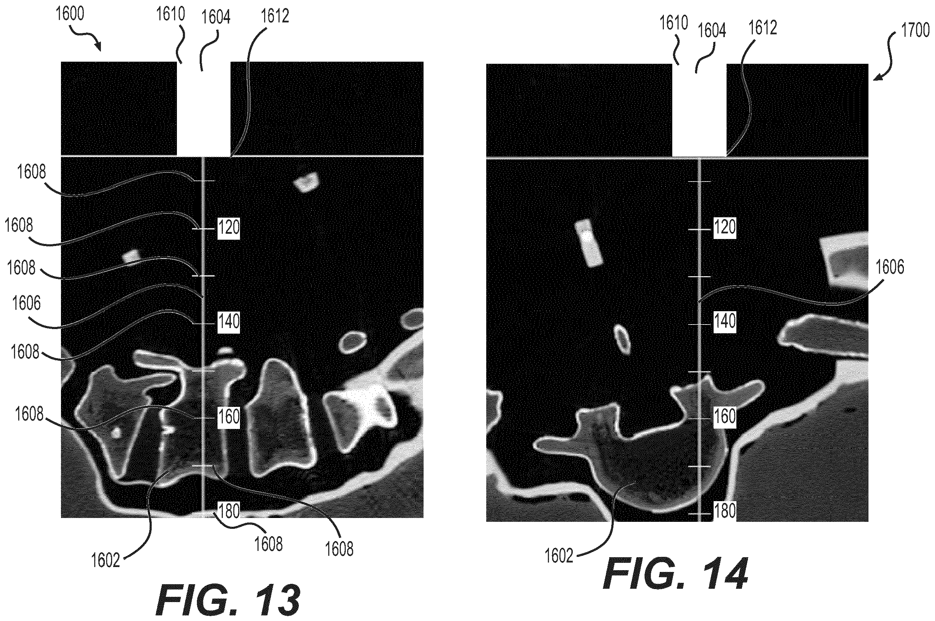

FIGS. 13-17 illustrate images and systems according to the invention for measuring the depth of surgical instrumentation during an operation on a patient. Implementations of the measurement systems and methods described may be conducted by the robot systems as previously described including robot systems 100, 300, and 600 and the components discussed thereto.

As an exemplary embodiment, a surgeon may position a long shaft such as a dilator tube within a secondary tracked tube, such as the robot's end effector guide tube or a free tracked external guide tube, to a known depth. These components have also been discussed as noted above. The depth may be the exact depth needed such that the dilator tube contacts the target bone of the patient. By referencing the secondary tracked tube, measuring the depth of the dilator tube may be conducted without tracking the dilator tube itself.

Depth measurements are based on the tracked guide tube-either the robot's end effector guide tube or other navigated guide tube-being in a "tube-centric" view. Viewplanes of various CT scans of a patient's target bone may be chosen such that the 3D-tracked position of the guide tube (for example, positional tracking as earlier discussed) is aligned to be simultaneously within two non-parallel planes, or in other words coincident with the line formed by the intersection of two planes. For example, in a posterior surgery where the guide tube is aligned such that it is aimed down a pedicle of a vertebral body, two substantially orthogonal anatomical viewplanes may be selected such that one viewplane is predominantly sagittal, with the guide tube aligned along the vertical axis of the 2D viewplane, while the other viewplane is predominantly axial, with the guide tube also aligned along that 2D viewplane's vertical axis. FIG. 13 illustrates an exemplary 2D image plane (slice) 1600 through a 3D computed tomography (CT) image volume showing vertebrae of a patient, including vertebral body 1602, a guide tube 1604, and vector 1606 from a sagittal viewpoint of the patient. FIG. 14 illustrates an exemplary 2D image plane (slice) 1700 through a 3D CT image volume showing vertebral body 1602, guide 1604, and vector 1606 from an axial view point of the patient.

Although FIGs. 13 and 14 show particular viewplanes, other pairs of viewplanes could be selected such that the tracked guide tube's central axis is coincident with the line found from the intersection of the two planes. In addition, the 2D representation on each plane of the tube does not need to be vertical or even in the same direction in the two planes.

Vector 1606 is depicted as extending from a representation of guide tube 1604 and overlaid on the images 1600 and 1700. On vector 1606, measurements may be shown incrementally with tick or hatch marks 1608. Marks 1608 may start from one end of guide tube 1604 (such as the top of proximal end 1610 and represented as position zero), with measurement increasing toward and beyond a distal end of the guide tube 1612. Guide tube 1604 is coincident with the viewplanes because it is then known that any anatomical point along vector 1606 is reachable by a tool inserted through guide tube 1606. If guide tube 1604 was not coincident with the viewplanes, a vector may still be drawn but the vector may a projection on the 2D view that may not intersect the anatomy visualized on the viewplane.

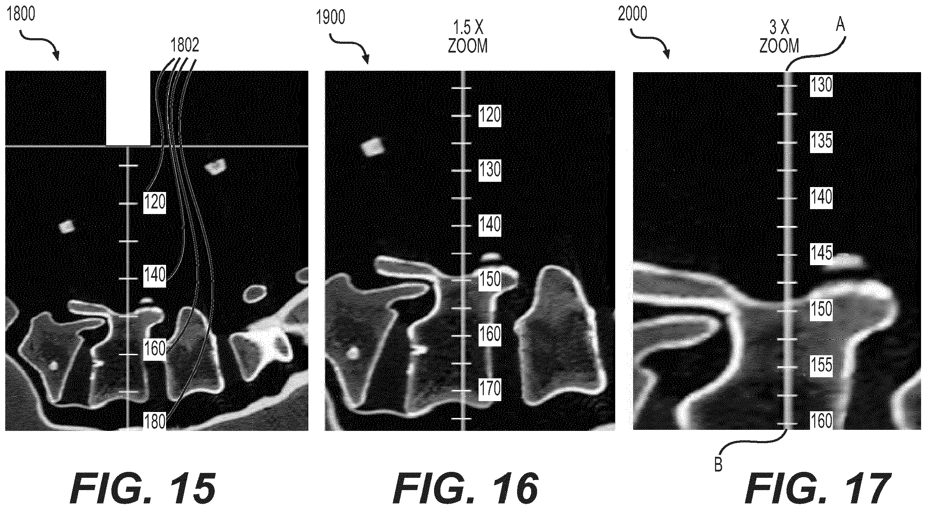

Marks 1608 on vector 1606 may be toggled on or off as needed. Additionally, as a user independently zooms in or out on a viewplane, the frequency and labeling of marks 1608 may be automatically modified to minimize clutter while still providing useful information to the user regarding the depth. For example, as shown in images 1800, 1900, and 2000 of FIGs. 15, 16, and 17, respectively, labels 1802 could be included at a rate of one label every 20 mm and marks 1606 at a rate of one hatch mark every 10 mm for a particular level of zoom (See Fig. 15). As zoom is increased to 1.5 times the original zoom (compare FIG. 16 to FIG. 15), labels 1802 could automatically switch to a rate of one label every 10 mm and marks 1606 could automatically switch to a rate of one hatch mark every 5 mm. Then, as zoom is increased to 3.0 times the original zoom (compare FIG. 17 to FIGs. 15 and 16), labels 1802 could automatically switch to a rate of one label every 5 mm and the marks 1606 could automatically switch to a rate of one hatch mark every 2.5 mm.

As an example, the decision for automatically switching rate and frequency of marks 1606 and labels 1802 could be that between two points A and B, marks 1606 may be present from the first to the last visible point on a line crossing the viewplane along the guide tube's central vector. Within points A and B, an optimal selection would involve increments representing well rounded values, such as tens, fives, ones, halves, and quarters. The frequency of labeling may be a rate of one label every C marks. For example, these rules would be able to dictate the behavior shown in FIGs. 15-17 if A=10, B=15, C=2. With zooming, the surgeon could read with good precision the distance from the top of the guide tube to the bone, for example 149 mm in FIGs. 15-17.

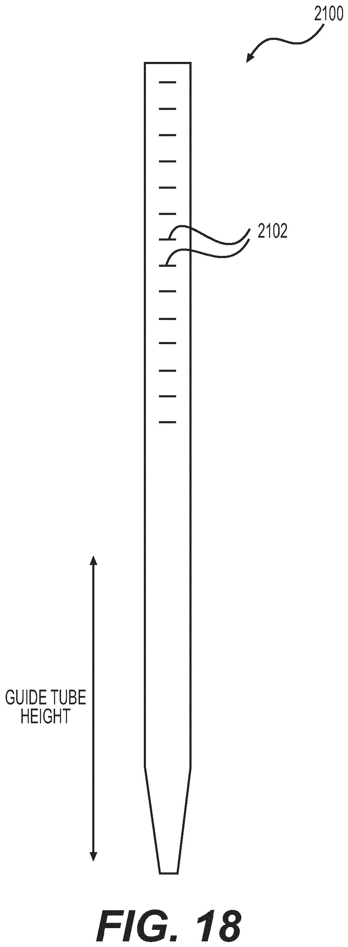

Using this measurement technique, the inserted instrument or dilator may have graduated markings and labels starting at zero at the distal end of the instrument and increasing toward the proximal end. FIG. 18 depicts an instrument 2100 with graduated markings 2102 showing this example. Markings 2102 may be present along the entire length of instrument 2100. In many cases, it may be unnecessary to include markings 2102 along the first length of instrument 2100 until the length of guide tube 1604 has been exceeded since markings 2102 would not be visible at the proximal end of guide tube 1604 with instrument 2100 inserted into guide tube 1604 and just beginning to protrude distally.

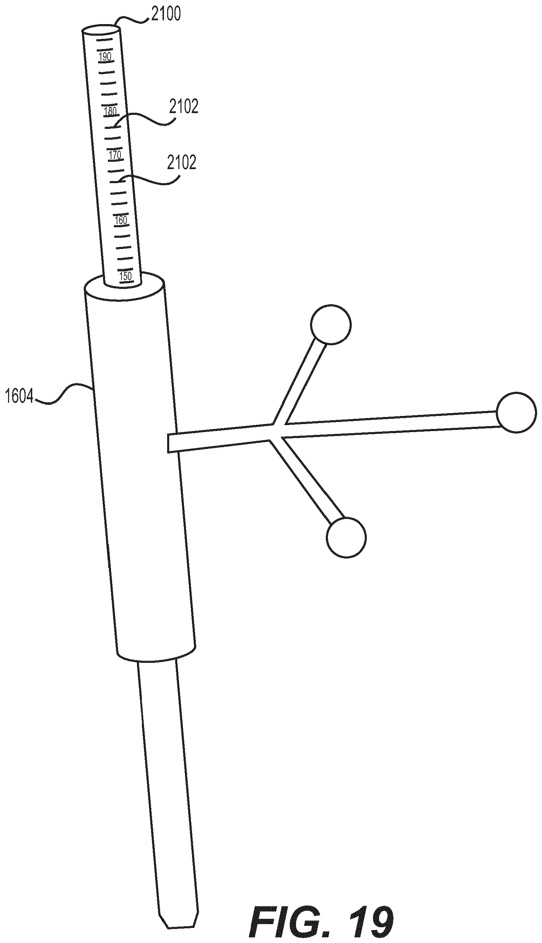

FIG. 19 illustrates instrument 2100, with markings 2102, inserted into guide tube 1604. The surgeon would insert instrument 2100 with markings into the tracked guide tube 1604 and would read the position of the inserted instrument 2100 from its graduations or markings 2102. This reading may indicate whether instrument 2100 reached the desired depth. For example, when inserting a dilator, if the length observed on markings 2102 agrees with a length measured from the measurement representing the distance from the proximal end of guide tube 1604 to the target bone of the patient, the surgeon would know that instrument 2100 was touching the target bone. If the length read from instrument 2100 was 3 mm less than the length from the measurement between the guide tube to the target bone, the surgeon would know that they must insert instrument 2100 a distance of 3 more millimeters before bone would be touched.

As described above, a distance from the top tip of instrument 2100 to the proximal end (top) of the tracked guide tube 1604 is being read from instrument 2100 by the use of markings 2102. Under the same principles of the present disclosure, measurement techniques may be configured such that the graphical images of the patient anatomy show the distance from the distal end (bottom) of tracked guide tube 1604, or the distance from a window through the guide tube 1604 to the tip of the instrument 2100. The insertion depth would then be read from these other locations, i.e., from the bottom of the guide tube or observed through a window in the guide tube, to draw the same information about the depth of insertion of the instrument.

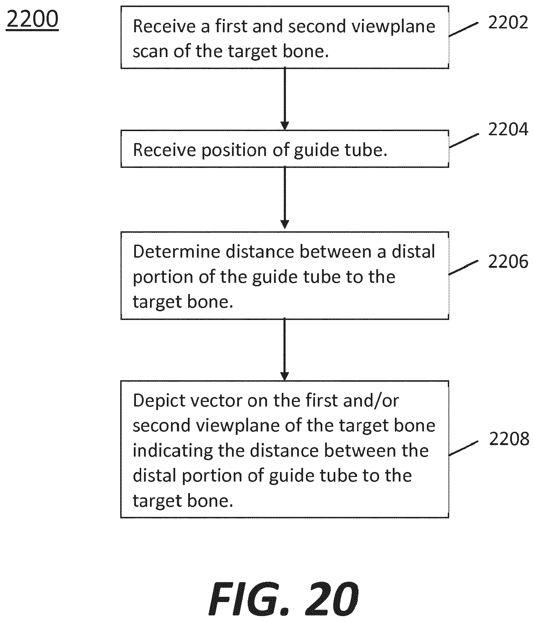

FIG. 20 illustrates a method 2200 for determining the distance or depth in order to contact a target bone of patient consistent with the principles of the present disclosure. At step 2202 a computer subsystem, such as those previously described, receives a scan, for example a CT scan, that may represent the target bone of a patient from different viewplanes or viewpoints. The computer subsystem may receive a scan of a first viewplane and a second viewplane that form an intersection of views of the target bone in an image space. At step 2204, the computer subsystem may receive information indicative of a position of a guide tube of the surgical robot system in navigational space. At step 2206 the computer of the computer subsystem may determine a distance between a distal portion of the guide tube to the target bone of the patient. The distance may be determined such that a central axis of the guide tube is coincident to the line of intersection formed between the first viewplane and the second viewplane. At step 2608 the computer subsystem may display a vector indicative of the distance to contact the target bone of the patient on at least one of the first viewplane scan and the second viewplane scan. The vector may include any or all of the attributes a previously describe and those skilled in the art would recognize that the visual depiction of the vector may be represented in other way to indicate distance measurements that would fall within the scope of the present disclosure.

While the invention has been disclosed in connection with the preferred embodiments shown and described in detail, various modifications and improvements thereon will become readily apparent to those skilled in the art. Accordingly, the scope of the present invention is not to be limited by the foregoing examples, but is is determined by the appended claims.

## Claims

1. A surgical robot system having a registration system (1400) for conducting registration procedures in order to track objects and a target bone of a patient (210) in both navigation space and an image space, the surgical robot system comprising:
- a tracked guide tube (1604) comprising a tracking marker (702) and wherein the tracked guide tube (1604) is configured to receive a surgical instrument (608);
- a tracking subsystem (532) having a position sensor (540) that recognizes the tracking marker (702) in a navigational space;
- a platform interface module configured to receive a signal from the tracking subsystem indicative of a position of the guide tube based on the tracking marker (702);
- a computer subsystem (408), including a computer and a display (110), configured to receive a first viewplane scan of a target bone and a second viewplane scan of the target bone, wherein the first viewplane and the second viewplane are so configured that an intersection of the first viewplane and the second viewplane defines an intersection line,
- wherein the computer subsystem (408) is configured to receive the position of the guide tube (1604) from the platform interface module (502), and
- wherein the computer subsystem (408), via the display (110), is configured to depict the tracked guide tube (1604) and a vector extending from the tracked guide tube (1604), indicative of the distance between a distal portion of the guide tube and the target bone of the patient, on both the first viewplane scan and the second viewplane scan, and
- wherein the view planes are selected such that the vector (1606) represents a central axis of the guide tube (1604) which is coincident to the intersection line,
- wherein the surgical robot system includes an end effector (602) for receiving the guide tube (1604), the end effector (602) includes tracking markers (702) disposed around the surface of the end effector (602) so that the end effector may be monitored by tracking devices when the end effector (602) is translated and rotated in the surgical field

2. The system of claim 1, wherein the vector (1606) comprises markings (1608) indicating the distance between the distal portion of the guide tube to the target bone.

3. The system of claim 2, wherein the markings (1608) may be depicted in rounded increments.

4. The system of claim 2, wherein the depiction of the vector (1606) may be zoomed in or out.

5. The system of claim 2, wherein increments of the markings (1608) automatically changes based upon a level of zoom.

6. The system of claim 1, further comprising the surgical instrument (608, 2100) received in the guide tube.

7. The system of claim 6, wherein the surgical instrument (608, 2100) contains graduated markings indicating the actual depth of the surgical instrument (608, 2100) past the distal portion of the guide tube (1604).

8. The system of claim 1, wherein the first viewplane is a sagittal view plane with respect to the target bone and the second viewplane is an axial viewplane with respect to the target bone.

9. The system of claim 1 wherein the guide tube is tracked separately from the end effector.

## Patentansprüche

1. Chirurgisches Robotersystem mit einem Registrierungssystem (1400) zum Durchführen von Registrierungsverfahren, um Objekte und einen Zielknochen eines Patienten (210) sowohl in einem Navigationsraum als auch in einem Bildraum nachzuverfolgen, wobei das chirurgische Robotersystem aufweist:
- ein nachverfolgtes Führungsrohr (1604) mit einer Nachverfolgungsmarkierung (702) und wobei das nachverfolgte Führungsrohr (1604) eingerichtet ist, um ein chirurgisches Instrument (608) aufzunehmen;
- ein Nachverfolgungssubsystem (532) mit einem Positionssensor (540), der die Nachverfolgungsmarkierung (702) in einem Navigationsraum erkennt;
- ein Plattformschnittstellenmodul, das eingerichtet ist, um ein Signal vom Nachverfolgungssubsystem zu empfangen, das eine Position des Führungsrohrs auf der Basis der Nachverfolgungsmarkierung (702) anzeigt;
- ein Computersubsystem (408), das einen Computer und eine Anzeige (110) umfasst, das eingerichtet ist, um eine erste Ansichtsebenenabtastung eines Zielknochens und eine zweite Ansichtsebenenabtastung des Zielknochens zu empfangen, wobei die erste Ansichtsebene und die zweite Ansichtsebene eingerichtet sind, so dass ein Schnittpunkt der ersten Ansichtsebene und der zweiten Ansichtsebene eine Schnittlinie definieren,
- wobei das Computersubsystem (408) eingerichtet ist, um die Position des Führungsrohrs (1604) vom Plattformschnittstellenmodul (502) zu empfangen, und
- wobei das Computersubsystem (408) über die Anzeige (110) eingerichtet ist, um das nachverfolgte Führungsrohr (1604) und einen Vektor darzustellen, der sich vom nachverfolgten Führungsrohr (1604) erstreckt, das den Abstand zwischen einem distalen Abschnitt des Führungsrohrs und dem Zielknochen des Patienten anzeigt, und zwar sowohl auf der ersten Ansichtebenenabtastung als auch auf der zweiten Ansichtebenenabtastung, und
- wobei die Ansichtsebenen ausgewählt werden, so dass der Vektor (1606) eine Mittelachse des Führungsrohrs (1604) darstellt, die mit der Schnittlinie übereinstimmt,
- wobei das chirurgische Robotersystem einen Endeffektor (602) zum Aufnehmen des Führungsrohrs (1604) umfasst, wobei der Endeffektor (602) Nachverfolgungsmarkierungen (702) umfasst, die um die Oberfläche des Endeffektors (602) angeordnet sind, so dass der Endeffektor durch Nachverfolgungsvorrichtungen überwacht werden kann, wenn der Endeffektor (602) im Operationsfeld verschoben und gedreht wird.

2. System nach Anspruch 1, wobei der Vektor (1606) Markierungen (1608) aufweist, die den Abstand zwischen dem distalen Abschnitt des Führungsrohrs zum Zielknochen anzeigen.

3. System nach Anspruch 2, wobei die Markierungen (1608) in gerundeten Inkrementen dargestellt werden können.

4. System nach Anspruch 2, wobei die Darstellung des Vektors (1606) vergrößert oder verkleinert werden kann.

5. System nach Anspruch 2, wobei sich Inkremente der Markierungen (1608) auf der Basis einer Zoomstufe automatisch ändern.

6. System nach Anspruch 1, das ferner das chirurgische Instrument (608, 2100), das im Führungsrohr aufgenommen ist, aufweist.

7. System nach Anspruch 6, wobei das chirurgische Instrument (608, 2100) abgestufte Markierungen enthält, die die tatsächliche Tiefe des chirurgischen Instruments (608, 2100) hinter dem distalen Abschnitt des Führungsrohrs (1604) anzeigen.

8. System nach Anspruch 1, wobei die erste Ansichtsebene eine sagittale Ansichtsebene in Bezug auf den Zielknochen und die zweite Ansichtsebene eine axiale Ansichtsebene in Bezug auf den Zielknochen ist.

9. System nach Anspruch 1, wobei das Führungsrohr getrennt vom Endeffektor nachverfolgt wird.

## Revendications

1. Système de robot chirurgical ayant un système d'enregistrement (1400) pour effectuer des procédures d'enregistrement afin de suivre des objets et un os cible d'un patient (210) à la fois dans un espace de navigation et dans un espace d'image, le système de robot chirurgical comprenant :
- un tube de guidage (1604) suivi comprenant un marqueur de suivi (702) et dans lequel le tube de guidage (1604) suivi est configuré pour recevoir un instrument chirurgical (608) ;
- un sous-système de suivi (532) ayant un capteur de position (540) qui reconnaît le marqueur de suivi (702) dans un espace de navigation ;
- un module d'interface de plate-forme configuré pour recevoir un signal du sous-système de suivi indicatif d'une position du tube de guidage sur la base du marqueur de suivi (702) ;
- un sous-système informatique (408), comportant un ordinateur et un dispositif d'affichage (110), configuré pour recevoir un premier balayage de plan de vue d'un os cible et un deuxième balayage de plan de vue de l'os cible, dans lequel le premier plan de vue et le deuxième plan de vue sont configurés de sorte qu'une intersection du premier plan de vue et du deuxième plan de vue définisse une ligne d'intersection,
- dans lequel le sous-système informatique (408) est configuré pour recevoir la position du tube de guidage (1604) du module d'interface de plate-forme (502), et
- dans lequel le sous-système informatique (408), via le dispositif d'affichage (110), est configuré pour présenter le tube de guidage (1604) suivi et un vecteur s'étendant depuis le tube de guidage (1604) suivi, indicatif de la distance entre une partie distale du tube de guidage et l'os cible du patient, à la fois sur le premier balayage de plan de vue et le deuxième balayage de plan de vue, et
- dans lequel les plans de vue sont sélectionnés de sorte que le vecteur (1606) représente un axe central du tube de guidage (1604) qui coïncide avec la ligne d'intersection,
- dans lequel le système de robot chirurgical comporte un effecteur final (602) pour recevoir le tube de guidage (1604), l'effecteur final (602) comporte des marqueurs de suivi (702) disposés autour de la surface de l'effecteur final (602) de sorte que l'effecteur final puisse être surveillé par des dispositifs de suivi lorsque l'effecteur final (602) réalise une translation et une rotation dans le champ chirurgical.

2. Système selon la revendication 1, dans lequel le vecteur (1606) comprend des marques (1608) indiquant la distance entre la partie distale du tube de guidage et l'os cible.

3. Système selon la revendication 2, dans lequel les marques (1608) peuvent être présentées par des incréments arrondis.

4. Système selon la revendication 2, dans lequel la présentation du vecteur (1606) peut être soumise à un zoom avant ou arrière.

5. Système selon la revendication 2, dans lequel des incréments des marques (1608) changent automatiquement sur la base d'un niveau de zoom.

6. Système selon la revendication 1, comprenant en outre l'instrument chirurgical (608, 2100) reçu dans le tube de guidage.

7. Système selon la revendication 6, dans lequel l'instrument chirurgical (608, 2100) contient des marques graduées indiquant la profondeur réelle de l'instrument chirurgical (608, 2100) au-delà de la partie distale du tube de guidage (1604).

8. Système selon la revendication 1, dans lequel le premier plan de vue est un plan de vue sagittal par rapport à l'os cible et le deuxième plan de vue est un plan de vue axial par rapport à l'os cible.

9. Système selon la revendication 1, dans lequel le tube de guidage est suivi séparément de l'effecteur final.
